# FASCICULE DE BREVET EUROPEEN

(11) **EP 3 234 070 B1**
(45) Date de publication et mention de la délivrance du brevet: **12.08.2020**
(21) Numéro de dépôt: 15820516.1
(22) Date de dépôt: 21.12.2015
(51) Int. Cl.: C10G 1/06, C10G 3/00

(54) **PROCEDE AMELIORE DE CONVERSION DE BIOMASSE ALGALE EN UN GAZ OU EN BIO-CRUDE RESPECTIVEMENT PAR GAZEIFICATION OU LIQUEFACTION HYDROTHERMALE**
VERBESSERTES UMWANDLUNGVERFAHREN VON ALGENBIOMASSE ZU EINEM GAS ODER BIO-ÖL DURCH BZW. HYDROTHERMALEN VERGASUNG ODER VERFLÜSSIGUNG
IMPROVED CONVERSION PROCESS OF ALGAE BIOMASS IN GAS OR BIO-CRUDE RESPECTIVELY BY HYDROTHERMAL GASIFICATION OR LIQUEFACTION

(30) Priorité: 19.12.2014 FR 1463026
(43) Date de publication de la demande: 25.10.2017
(73) Titulaire: Commissariat à l'Énergie Atomique et aux Énergies Alternatives, 75015 Paris (FR)
(72) Inventeur: TEXIER, Jonathan, 17150 Nieul le Virouil (FR); CASTELLI, Pierre, 38330 Saint-Nazaire les Eymes (FR); ROUBAUD, Anne, 38690 Chabons (FR)
(74) Mandataire: Nony
(86) Numéro de dépôt international: PCT/EP2015/080854
(87) Numéro de publication internationale: WO 2016/097414

(56) Documents cités:
- US-A- 4 338 199
- US-A- 5 106 513
- US-A1- 2012 210 632
- US-A1- 2014 273 141
- SAMUEL STUCKI ET AL: "Catalytic gasification of algae in supercritical water for biofuel production and carbon capture", ENERGY & ENVIRONMENTAL SCIENCE, vol. 2, no. 5, 1 janvier 2009 (2009-01-01) , page 535, XP055205004, ISSN: 1754-5692, DOI: 10.1039/b819874h
- JOHN J. MILLEDGE ET AL: "Methods of energy extraction from microalgal biomass: a review", REVIEWS IN ENVIRONMENTAL SCIENCE AND BIO-TECHNOLOGY, vol. 13, no. 3, 1 septembre 2014 (2014-09-01), pages 301-320, XP055255316, NL ISSN: 1569-1705, DOI: 10.1007/s11157-014-9339-1
- MAN KEE LAM ET AL: "Microalgae biofuels: A critical review of issues, problems and the way forward", BIOTECHNOLOGY ADVANCES., vol. 30, no. 3, 1 mai 2012 (2012-05-01), pages 673-690, XP055255323, GB ISSN: 0734-9750, DOI: 10.1016/j.biotechadv.2011.11.008

## Description

### Domaine technique

La présente invention concerne un procédé amélioré de conversion thermochimique de biomasse algale, avantageusement intégré en continu au sein d'un procédé de culture d'algues.

L'invention vise à améliorer la conversion de la biomasse algale en un gaz ou en bio-crude respectivement par gazéification hydrothermale ou liquéfaction hydrothermale en vue de la production de bio-carburants (liquides ou gazeux).

Par «bio-crude», il est entendu le sens usuel, c'est-à-dire un liquide combustible issu de la liquéfaction hydrothermale directe de la biomasse algale.

Par « biomasse algale », il est entendu l'ensemble des micro-organismes photo synthétiques.

Par « micro-organismes photosynthétiques», il est entendu le sens usuel, tel que donné dans la publication [1], à savoir des algues et des cyanobactéries, *i.e.* des organismes eucaryotes ou procaryotes dont la croissance s'effectue principalement par photosynthèse, et de taille microscopique, typiquement comprise entre 1 et 100 µm.

Par « eau supercritique », il est entendu le sens usuel, c'est-à-dire de l'eau à des températures supérieures à 374°C sous une pression supérieure à 22,1 MPa.

### Etat de la technique

A l'état naturel, les micro-organismes photosynthétiques (micro-algues et cyanobactéries), sont des organismes photosynthétiques unicellulaires, principaux composants du phytoplancton, qui peuplent les océans et cours d'eau depuis plus de trois milliards et demi d'années.

La culture des micro-algues et des cyanobactéries à l'échelle industrielle connaît depuis peu un fort engouement du fait des nombreux champs d'application parmi lesquelles, dans une échelle de valorisation décroissante, la pharmaceutique et la cosmétique, l'alimentation animale, l'alimentation humaine, les fertilisants, les matériaux biosourcés, la bio-rémédiation et les bio-carburants (liquides ou gazeux).

Le fort engouement de cette culture dédiée provient essentiellement du fait qu'elle ne nuit pas aux productions alimentaires, et que son rendement, c'est-à-dire les taux de croissance des micro-algues et cyanobactéries, peut être très élevé. Pour donner un ordre de grandeur, comme avancé par certains auteurs, un hectare de culture de micro-algues peut théoriquement produire entre 60 et 300 barils équivalent pétrole par an, contre 7 barils avec du colza. Pour permettre la croissance des micro-algues ou des cyanobactéries, il faut combiner une souche avec le milieu de culture adéquat. Ce dernier se compose d'eau, de nutriments, de dioxyde de carbone (CO₂) et de l'énergie solaire. Du dosage de ces différents paramètres vont découler le taux de multiplication et de croissement de la biomasse algale ainsi que sa composition. Ainsi, par exemple une carence en azote dans le milieu de culture peut conduire à une accumulation de lipide au sein des cellules.

Les trois espèces de micro-organismes photosynthétiques actuellement les plus cultivées sont, par ordre de quantité décroissante, les cyanobactéries du genre *Arthrospira* plus connue sous l'appellation de « spiruline », qui représente environ 50% de la production mondiale, suivie par les micro-algues vertes des genres *Chlorella*, *Dunaliella, Haematococcus, Nannochloropsis* et les diatomées du genre *Odontella.*

De manière générale, en dehors de quelques espèces cultivées pour des marchés de niche, les méthodes de culture des micro-organismes photosynthétiques n'ont pas encore atteint leur niveau de maturité industrielle. Des problématiques de constance de qualité et de leur coût de production se posent encore, ce qui limite leur accès à de nombreux marchés commerciaux. En outre, la production de micro-organismes photosynthétiques nécessite de la lumière ainsi que de la chaleur pendant les périodes froides. Or, pour ne pas la limiter aux seules régions chaudes et ensoleillées, les solutions techniques retenues doivent permettre d'augmenter la productivité des micro-algues dans des pays situés aux latitudes des régions tempérées.

Les systèmes de production par monoculture de micro-organismes photosynthétiques en suspension dans l'eau sont nombreux.

Deux grandes familles de systèmes de culture [1] des micro-organismes photosynthétiques (biomasse algale), se distinguent notamment :
- les systèmes d'architecture de type ouverte, qui sont constitués par des bassins, le cas échéant en boucle également connus sous la dénomination « Raceway », lagunes, récipients à ciel ouvert, c'est-à-dire à l'air libre. Ils ont comme avantage principal de générer des coûts de production relativement faibles. Leurs inconvénients majeurs résident dans une productivité volumique faible, c'est-à-dire des tailles d'équipements requis qui sont importants pour une production de biomasse donnée, et une mauvaise maitrise des conditions de culture (sensibilité aux contaminations notamment),
- les systèmes d'architecture de type fermée, connus sous l'appellation « photo-bioréacteurs », constitués par une ou plusieurs enceinte(s), c'est-à-dire dont l'intérieur n'est pas en contact avec l'air ambiant. Bien que nécessitant un investissement initial important, les photo-bioréacteurs permettent comparativement à ceux ouverts, d'atteindre de meilleures productivités et de réaliser des cultures dans des conditions mieux maitrisées, en évitant notamment les contaminations, pollutions, et les pertes de CO₂.

La liquéfaction hydrothermale est un procédé de conversion prometteur pour transformer la biomasse algale en bio-carburants dits de 3^{ème} génération. Il s'agit d'un procédé opérant en eau sous critique, c'est-à-dire dans des conditions de température et de pression inférieures à 374°C et 22,1MPa, dans lesquelles l'eau joue un rôle à la fois de milieu de réaction et de réactif. En première approche, ce procédé permet de transformer une biomasse algale, encore en solution dans son milieu de culture, en un bio-crude, beaucoup plus proche d'un carburant liquide.

Cependant, un inconvénient majeur de ce procédé de conversion par liquéfaction hydrothermale est la production d'un effluent aqueux lors de la réaction. Ce dernier contient les nutriments nécessaires à la croissance des algues, mais également des composés carbonés, pour certains toxiques pour les algues.

Ainsi, à l'échelle de laboratoire, bon nombre de personnes ont déjà démontré la faisabilité d'une conversion de la biomasse algale par liquéfaction hydrothermale.

En particulier, la demanderesse a déjà montré que la liquéfaction hydrothermale est un procédé envisageable pour la conversion d'une multitude d'algues (*Nannochloropsis, Chlorelle, Néochloris, Spiruline, Chlamydomonas, Dunaliella, etc.*) encore dans leur milieu de culture, en un bio-crude combustible.

La publication [2] met en évidence l'étude de différentes températures et temps de séjours de la biomasse algale dans un réacteur de mise en œuvre de la liquéfaction hydrothermale. Cette publication [2] montre que des temps de séjours courts, typiquement de 1 à 5 minutes, suffisent pour obtenir des taux de conversion en bio-crude élevés, allant jusqu'à 66%, et des valeurs de pouvoir calorifique supérieur (PCS) jusqu'à 36 MJ/kg. Dans cette publication [2], le recyclage des effluents en sortie de procédé n'est pas considéré.

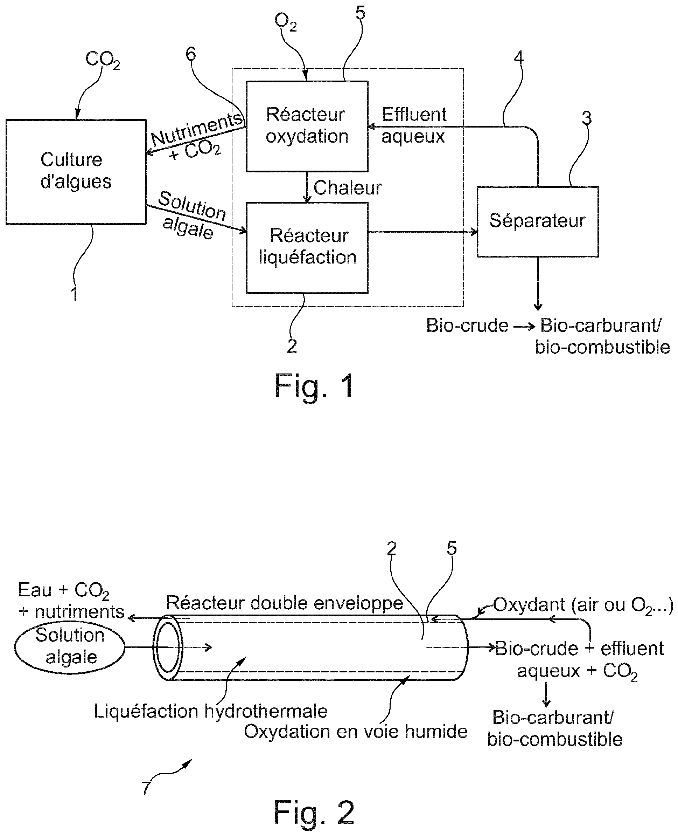

La publication [3] s'intéresse à la liquéfaction hydrothermale de déchets aqueux, et d'une biomasse algale. Dans cette publication [3], il est envisagé le recyclage des nutriments et du carbone issus du milieu de culture des algues : le schéma de recyclage envisagé est représenté en figure 1 de la publication. Dans cette publication [3], le résidu de la liquéfaction hydrothermale n'est cependant pas retraité et sert directement à cultiver des algues, ce qui peut s'avérer néfaste et non optimal pour la culture, car certains composés issus de la liquéfaction hydrothermale sont toxiques.

De manière plus générale, les différentes équipes travaillant sur la liquéfaction hydrothermale publient des résultats similaires et très encourageants, en termes de taux de conversion en bio-crude, et de pouvoir calorifique du bio-crude obtenu.

En d'autres termes, la faisabilité de transformer une biomasse algale, voire des déchets de types agro-alimentaire, en bio-crude est maintenant avérée, et le niveau des performances atteignables par un procédé continu de liquéfaction hydrothermale est appréhendé.

La demande de brevet US 2012/0055077 propose une amélioration du procédé de liquéfaction hydrothermale d'une biomasse par un post-traitement du bio-crude en sortie de liquéfaction hydrothermale pour en améliorer ses propriétés de bio-carburant. Cette demande de brevet ne s'intéresse pas au retraitement de la phase aqueuse issue de la liquéfaction.

La demande de brevet WO 2011/049572 vise à proposer un procédé de liquéfaction hydrothermale d'un déchet, et de valoriser le CO₂ obtenu pour la culture d'algues, la biomasse algale ainsi obtenue étant à son tour convertie par liquéfaction hydrothermale. Dans le procédé divulgué, la phase aqueuse en sortie de liquéfaction hydrothermale n'est que très peu valorisée. En effet, celle-ci est simplement réinjectée directement dans le milieu de culture des algues, ce qui peut s'avérer toxique pour les algues et non optimal pour leur croissance.

La demande de brevet US 2012/0285077 propose quant à elle une extraction de type biologique des lipides contenus dans des algues afin d'obtenir une bio-huile, ensuite convertie en biodiesel par mise en œuvre d'une réaction de transestérification. Le procédé divulgué dans cette demande envisage le recyclage des résidus par fermentation pour former du CO₂ qui est ensuite réinjecté dans un réacteur de culture d'algues.

La demande de brevet US2014/273141 divulgue un procédé de conversion de la biomasse algale par liquéfaction hydrothermale.

Ainsi, bien que de nombreuses équipes travaillent au développement du procédé de conversion de la biomasse algale en bio-crude par liquéfaction hydrothermale, et que ce procédé implique la production importante d'effluents aqueux lors de la réaction, aucune solution n'a réellement été proposée pour utiliser au mieux ces effluents aqueux.

Il existe un besoin pour améliorer les procédés de conversion thermochimique de biomasse algale en gaz ou en bio-crude en vue de la production de bio-carburants (liquides ou gazeux), notamment en vue d'utiliser au mieux les effluents aqueux produits lors de ces procédés.

Plus généralement, il existe un besoin pour améliorer les procédés en continu qui intègrent un procédé de conversion thermochimique de biomasse algale en gaz ou en bio-crude en aval d'un procédé de culture de la biomasse algale.

Le but général de l'invention est de répondre au moins en partie à ce(s) besoin(s).

### Exposé de l'invention

Pour ce faire, l'invention a tout d'abord pour objet, selon un aspect, un procédé de conversion de la biomasse algale, c'est-à-dire l'ensemble des micro-organismes photosynthétiques, en un gaz ou en un bio-crude en vue de produire un combustible ou un carburant, notamment un carburant liquide, ou un autre produit de synthèse, comprenant les étapes suivantes :
a/ gazéification hydrothermale ou liquéfaction hydrothermale d'une biomasse algale dans au moins un premier réacteur,
b/ séparation entre respectivement le gaz ou le bio-crude produit, et les effluents aqueux et le CO₂ produit, en sortie du premier réacteur,
c/ récupération des effluents aqueux,
d/ oxydation des effluents aqueux dans au moins un deuxième réacteur.

l'étape d/ étant une oxydation par voie humide ou une oxydation hydrothermale (OHT) en conditions supercritiques,
la chaleur produite lors de l'étape d/ d'oxydation dans le deuxième réacteur étant apportée dans le premier réacteur pour la mise en œuvre de l'étape a/ de liquéfaction ou gazéification hydrothermale.

Ainsi, l'invention permet en premier lieu à la fois de produire du gaz ou du bio-crude et de transformer efficacement les effluents aqueux néfastes afin de les recycler. Les effluents aqueux ainsi transformés ne constituent plus des déchets.

Plus précisément, en sortie du premier réacteur il peut être récupéré un gaz ou du bio-crude directement utilisable pour produire des carburants liquides, et en sortie du second réacteur, il peut être récupéré et valorisé de l'eau et des nutriments ainsi que le CO₂ produit lors de la gazéification ou la liquéfaction hydrothermale.

L'invention a également pour objet, selon un autre de ses aspects, un procédé en continu de culture de biomasse algale et de conversion de la biomasse algale cultivée en un gaz ou en un bio-crude, comprenant les étapes suivantes :
- culture dans une zone de culture contenant un milieu de culture la biomasse algale à cultiver,
- récolte de la biomasse algale cultivée,
- étapes a/ à d/ du procédé de conversion décrit précédemment,
- injection de l'eau et des nutriments, et le cas échéant du CO₂, obtenus en sortie du deuxième réacteur, dans la zone de culture.

Selon un mode de réalisation avantageux, le procédé en continu, comprend en outre l'étape de récupération de l'oxygène (O₂) produit par la biomasse algale pour l'injecter, en tant qu'oxydant, en amont du deuxième réacteur avant l'étape d/ d'oxydation.

Ainsi, l'invention propose d'intégrer un recyclage des effluents produits, au sein même du procédé en continu, grâce à la mise en place de deux réacteurs, l'un dédié la gazéification ou la liquéfaction hydrothermale pour la valorisation de la biomasse algale en carburant et l'autre dédié à l'oxydation des effluents aqueux pour permettre leur recyclage dans le milieu de culture de la biomasse algale.

Lorsque les deux réacteurs sont couplés thermiquement, l'énergie libérée par la réaction exothermique d'oxydation peut être valorisée au mieux, en l'apportant comme source de chaleur à la réaction endothermique de gazéification ou de liquéfaction hydrothermale.

Ainsi, les avantages procurés par l'invention sont nombreux parmi lesquels :
- une économie sur le retraitement du CO₂ et des effluents aqueux en sortie de procédé de conversion de la biomasse algale en gaz ou en bio-crude, ces effluents aqueux ne constituant plus des déchets qu'il faut retraiter comme selon l'état de l'art.
- la récupération d'un milieu de culture non toxique (eau et nutriments en sortie de réacteur d'oxydation) pour la culture de la biomasse algale, et donc une économie de production pour la cultiver,
- un apport d'énergie pour la réaction endothermique de liquéfaction hydrothermale par la réaction exothermique d'oxydation.

Lorsque l'étape a/ est une liquéfaction, elle est réalisée de préférence dans les gammes de températures comprises entre 150 et 374°C et/ou de pressions comprises entre 0,5 et 35 MPa.

Lorsque l'étape a/ est une gazéification, elle est réalisée de préférence dans une gamme de températures comprises entre 374 et 800°C et/ou de pressions comprises entre 22,1 et 35 MPa.

De préférence, l'étape a/ est réalisée dans une gamme de temps de séjour compris entre 15 secondes et 1 heure.

Lorsque l'étape d/ est une oxydation par voie humide réalisée dans une gamme de températures comprises entre 150 et 374°C et/ou de pressions comprises entre 0,5 et 35 MPa.

Lorsque l'étape d/ est une oxydation hydrothermale réalisée dans une gamme de températures comprises entre 374 et 800°C et/ou de pressions comprise entre 22,1 et 35 MPa.

L'étape d/ est de préférence, réalisée dans une gamme de temps de séjour dans le deuxième réacteur compris entre 15 secondes et 1 heure.

Selon une variante avantageuse, l'étape d/ est une oxydation par voie humide réalisée avec une partie du bio-crude produit injecté dans le deuxième réacteur.

Le CO₂ produit et séparé peut être injecté, en tant que solvant, dans le bio-crude produit.

La chaleur produite lors de l'étape d/ d'oxydation dans le deuxième réacteur est apportée dans le premier réacteur pour la mise en œuvre de l'étape a/ de liquéfaction ou gazéification hydrothermale.

La chaleur produite lors de l'étape d/ d'oxydation dans le deuxième réacteur peut également être apportée pour préchauffer le gaz oxydant.

Selon un mode de réalisation avantageux,_l'étape a/ de liquéfaction hydrothermale et l'étape d/ d'oxydation sont réalisées au sein d'un même réacteur, dit à double enveloppe, comprenant une enveloppe interne délimitant intérieurement la chambre du premier réacteur, et une enveloppe externe entourant l'enveloppe interne, l'espace entre l'enveloppe interne et l'enveloppe externe définissant la chambre du deuxième réacteur.

Avantageusement, l'étape a/ de liquéfaction ou gazéification hydrothermale est réalisée dans plusieurs premiers réacteurs en parallèle fluidique.

Avantageusement encore, l'étape d/ d'oxydation est réalisée dans plusieurs deuxièmes réacteurs en parallèle fluidique.

L'invention a également pour objet un procédé en continu de culture de biomasse algale et de conversion de la biomasse algale cultivée en un gaz ou en un bio-crude, comprenant les étapes suivantes :
- culture de la biomasse algale dans une zone contenant un milieu de culture
- récolte de la biomasse algale cultivée,
- étapes a/ à d/ du procédé de conversion selon l'une des revendications précédentes,
- injection de l'eau et des nutriments, et le cas échéant du CO₂, obtenus en sortie du deuxième réacteur, dans la zone de culture.

Le procédé peut comprendre en outre l'étape de récupération de l'oxygène (O₂) produit par la biomasse algale pour l'injecter, en tant qu'oxydant, en amont du deuxième réacteur avant l'étape d/ d'oxydation.

L'invention a encore pour objet l'utilisation du procédé de conversion décrit ci-dessus ou du procédé en continu décrit ci-dessus pour la production de carburants liquides, dits de 3^{ème} génération.

### Description détaillée

D'autres avantages et caractéristiques de l'invention ressortiront mieux à la lecture de la description détaillée de l'invention faite à titre illustratif et non limitatif en référence aux figures suivantes parmi lesquelles :
- la figure 1 est une vue schématique d'un exemple de système mettant en œuvre le procédé en continu de culture de biomasse algale et de conversion de la biomasse algale cultivée par liquéfaction hydrothermale en bio-crude selon l'invention;
- la figure 2 est une vue schématique en perspective d'un mode de réalisation avantageux d'une partie du système selon l'invention avec réacteur à double enveloppe;
- la figure 3 est une vue schématique d'un exemple de système mettant en œuvre un procédé en continu de culture de biomasse algale et de conversion de la biomasse algale cultivée par liquéfaction hydrothermale en bio-crude selon l'état de l'art;
- la figure 4 est une vue schématique d'un exemple de système mettant en œuvre un procédé en continu selon l'invention et directement appliqué au système selon la figure 3;
- la figure 5 est un graphe indiquant les gammes préférées de température et de pression pour la réalisation de l'étape a/ de liquéfaction hydrothermale dans le procédé selon l'invention.

Dans la description qui va suivre les termes « entrée », « sortie » « amont », « aval », sont utilisés par référence avec la direction de circulation des produits obtenus au sein du système mettant en œuvre le procédé en continu selon l'invention.

La mention X%massique signifie un pourcentage X en masse d'un composé.

Sur la figure 1, est représenté schématiquement un premier exemple d'un système mettant en œuvre le procédé continu de culture de micro-organismes photosynthétiques et leur conversion par liquéfaction hydrothermale selon la présente invention.

Le système comporte tout d'abord une zone 1 de culture de la biomasse algale. Cette zone de culture est soit à ciel ouvert, avec un ou plusieurs bassins, soit de type fermé, avec un ou plusieurs photo-bioréacteurs (PBR).

Le bassin à ciel ouvert peut être de type à boucle(s), comme appelé usuellement « raceway ».

Pour la culture des espèces de micro-algues et cyanobactéries ci-après, les gammes préférentielles de température suivantes peuvent être envisagées :
- *Arthrospira platensis* : 25 - 35°C (température optimale = 30°C),
- *Chlorella pyrenoidosa* : 35 - 45°C (température optimale = 38,7°C),
- *Chlorella vulgaris* : 25 - 35°C (température optimale = 30°C),
- *Chlamydomonas reinhardtii* : 15 - 30°C (température optimale = 25°C),
- *Phaeodactylum tricornutum* : 20 - 25°C (température optimale = 22,5°C),
- *Porphyridium cruentum* : 15 - 30°C (température optimale = 19,1°C),
- *Scenedesmus sp.* : 20 - 33°C (température optimale = 26,3°C),
- *Nannochloropsis oceanica* : 20 - 33°C (température optimale = 26,7°C),
- *Dunaliella tertiolecta* : 30 - 39°C (température optimale = 32,6°C).

La biomasse algale obtenue par la culture est alors récoltée.

Différentes techniques peuvent être utilisées pour la récolte, telles que la floculation, la filtration, la centrifugation.

L'étape ultérieure de liquéfaction hydrothermale présente l'avantage de fonctionner avec un taux d'humidité élevé, jusqu'à 80%massique d'eau, voire plus. Contrairement à d'autres technologies de conversion, cela permet de simplifier les étapes de récolte et de séchage.

La biomasse récoltée peut être envoyée, notamment au moyen d'une pompe d'aspiration 10 vers un réacteur 2 de transformation de la biomasse algale en bio-crude (phase huileuse) par liquéfaction hydrothermale. Outre le bio-crude en sortie du réacteur 2, il se forme une phase aqueuse, contenant des résidus organiques et des nutriments du milieu de culture, et une phase gazeuse, contenant en grande majorité du CO₂. Autrement dit, la réaction de liquéfaction hydrothermale, qui est endothermique, permet la transformation d'une solution algale, partiellement concentrée dans son milieu de culture, en bio-crude d'intérêt, en un effluent aqueux, et en CO₂. Le bio-crude obtenu peut alors subir un post-traitement, de l'hydro-liquéfaction par exemple, pour être transformé en bio-carburant type bio-diesel.

Selon l'invention, en sortie de réacteur 2, il est réalisé une séparation entre les effluents aqueux, le CO₂, et le bio-crude au moyen d'un dispositif 3 approprié, qui peut être usuel.

Puis, les effluents aqueux, de préférence avec le CO₂, sont envoyés par une ligne de retour vers l'entrée d'un réacteur 5 dans lequel une réaction d'oxydation par voie humide va avoir lieu.

La réaction d'oxydation, qui est une réaction exothermique, permet de transformer les effluents aqueux, obtenus avec la réaction de liquéfaction dans le réacteur 1, en eau contenant les nutriments présents dans la solution algale initiale, et en CO₂. Pour avoir lieu, cette réaction nécessite un oxydant, qui peut être de l'air, de l'oxygène, ou autre. Pour que la réaction d'oxydation en voie humide soit viable énergétiquement, il est préférable que la séparation des phases en sortie de liquéfaction se fasse sans refroidir ni dépressuriser le mélange.

Puis, en sortie de réacteur, un mélange de nutriments avec de l'eau et éventuellement du CO₂ est obtenu, qui peut être injecté via la ligne 6 dans la zone de culture 1. Ce mélange est donc recyclable dans la culture d'algues.

Ainsi, selon l'invention, le traitement des effluents aqueux, en sortie de liquéfaction hydrothermale, est réalisé grâce à l'oxydation, pour la culture de nouvelles algues.

L'invention décrite permet en outre de valoriser au mieux l'énergie.

En effet, la réaction de liquéfaction nécessite de chauffer la solution algale et est endothermique, alors que la réaction d'oxydation, quant à elle, est exothermique.

Ainsi, comme schématise en figure 1, la chaleur issue de la réaction exothermique d'oxydation par voie humide qui a lieu dans le réacteur 5 est apportée au réacteur 2 pour réaliser ou participer à la liquéfaction hydrothermale.

Ainsi, pour combiner ces deux réactions, il peut être utilisé avantageusement un réacteur 7 dit à double enveloppe comme illustré en figure 2. Dans ce réacteur 7, l'enveloppe interne délimite la chambre du réacteur 2 dans laquelle la réaction de liquéfaction hydrothermale a lieu et l'espace entre l'enveloppe interne et celle externe délimite la chambre du réacteur 5 dans laquelle a lieu la réaction d'oxydation par voie humide (ou inversement).

La structure et le fonctionnement d'un tel réacteur 7 à double enveloppe est maintenant expliqué.

Le réacteur 7 est tubulaire et sa longueur permet d'avoir une surface de contact suffisante entre les deux zones réactionnelles, et ainsi améliorer les échanges thermiques.

De plus, un diamètre réduit, permet de minimiser l'épaisseur nécessaire pour contenir la pression interne. L'enveloppe externe doit résister aux contraintes mécaniques : la pression et la température, et aux contraintes chimiques liées à l'oxydation de matière organique et aux ions issus des sels minéraux. Typiquement, l'enveloppe externe du réacteur 7 peut être en acier 316L ou Inconel 625®. Si nécessaire, pour améliorer la tenue chimique du réacteur, un chemisage en titane peut être envisagé.

L'enveloppe interne est utilisée en équi-pression entre les deux zones réactionnelles, elle n'a donc pas à résister à la pression. Elle doit cependant résister aux contraintes thermiques, assurer une bonne conductivité thermique, et résister aux contraintes chimiques des deux réactions, *i.e.* à la fois de liquéfaction dans la chambre 2 et celle d'oxydation dans la chambre 5. Typiquement, il peut être envisagé une enveloppe interne en titane, qui peut résister aux contraintes chimiques et thermiques, mais possède une conductivité thermique réduite. De l'acier 316L ou de l'Inconel 625® peuvent également être mis en œuvre.

Au lieu d'utiliser un réacteur à double enveloppe 7, il est également possible d'utiliser d'autres systèmes d'échangeurs de chaleur (échangeurs à tubes, à faisceau, à spirales, *etc.*) ou encore valoriser l'énergie produite par la réaction d'oxydation autrement.

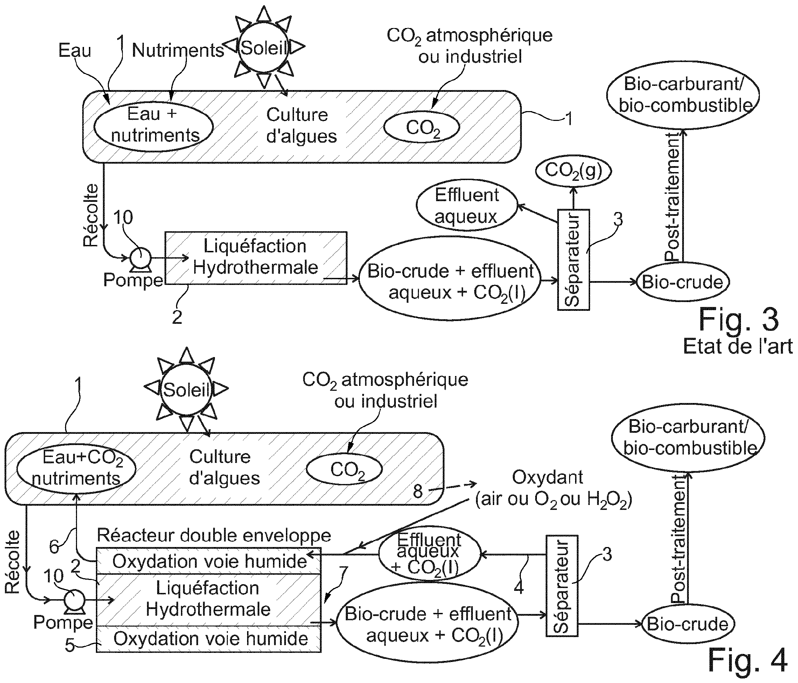

Il est représenté en figure 3 un système mettant en œuvre un procédé en conversion en continu de culture de biomasse algale et de conversion de la biomasse algale cultivée par liquéfaction hydrothermale en bio-crude selon l'état de l'art.

Dans un tel système, les effluents aqueux et le CO₂ produits sont des déchets gênants.

Pour pallier à cet inconvénient, les inventeurs ont ainsi proposé le procédé déjà décrit ci-dessus avec un recyclage optimisé :
- par l'oxydation des effluents aqueux récupérés en sortie du dispositif de séparation 3,
- puis, par l'injection d'un flux composé d'eau, de CO₂ et de nutriments produits par l'oxydation dans la zone de culture 1.

Pour réaliser la réaction d'oxydation par voie humide, il est nécessaire d'ajouter un oxydant au mélange. Ce dernier peut être de l'air, de l'oxygène, du peroxyde d'hydrogène, ou autres. Avantageusement, il est possible de récupérer l'O₂ produit lors de la croissance des algues dans la zone de culture pour l'injecter avant l'étape d'oxydation en voie humide, c'est-à-dire en entrée du réacteur 5 comme symbolisé en figure 4.

Ainsi, grâce à l'invention, les coproduits de la réaction de liquéfaction hydrothermale qui sont gênants dans le système selon l'état de l'art sont valorisés au sein même du procédé de culture de la biomasse algale.

Avec l'étape supplémentaire d'oxydation selon l'invention, le rendement énergétique du procédé de liquéfaction hydrothermale est augmenté, et le retraitement et la valorisation des effluents gênants en sortie de procédé deviennent possibles.

Il est précisé ci-après les paramètres et conditions préférés de mise en œuvre des différentes étapes du procédé selon l'invention.

### Etape de liquéfaction hydrothermale

### i. Produits en entrée

En entrée de réacteur 2, peuvent être injectés différents types d'algues (*Nannochloropsis, Chlorelle, Néochloris, Spiruline, Chlamydomonas, Dunaliella, etc.*) plus ou moins concentrées dans leur milieu de culture.

Pour effectuer un essai de liquéfaction, la solution algale présente avantageusement une concentration entre 10%_{massique} et 40%_{massique} de matière sèche (matière organique et inorganique), sachant que la composition élémentaire chimique de la matière sèche est comprise dans les gammes des valeurs suivantes :
- 20 à 70%_{massique} de carbone
- 5 à 40%massique d'oxygène
- 5 à 10%massique d'hydrogène
- 5 à 10%_{massique} d'azote
- 3 à 50%massique de sels inorganiques (composés de P, K, Cl, Na, S, Mg, Ca, Fe, Al, F, *etc.*)*.*

Par la suite, il est considéré une concentration intermédiaire de 20%_{massique} de matière sèche composée comme suit : 55%massique de carbone ; 25%massique d'oxygène ; 7%massique d'hydrogène, 8%massique d'azote et 5%massique de sels. Cette composition (C_{6,0}H_{9,1}O_{2,0}N_{0,7}) correspond à un pouvoir calorifique supérieur (PCS) de la matière sèche d'environ 24,5 MJ/kg.

### ii. Conditions opératoires

La température de réaction est comprise entre 200 à 350°C pour des pressions variant entre 5 à 25 MPa.

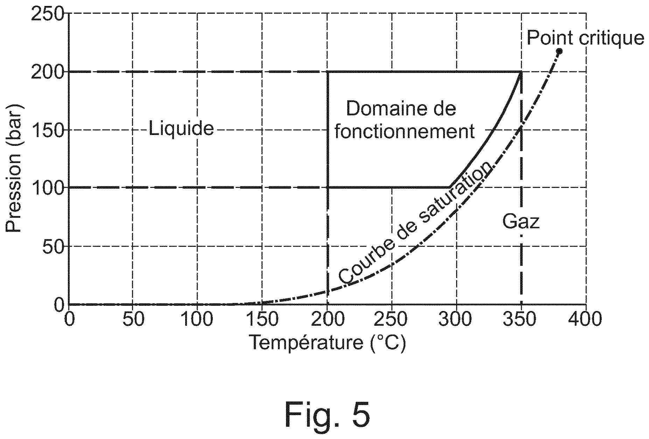

Typiquement, pour que la réaction de liquéfaction ait lieu, les conditions de température et de pression doivent se situer dans le domaine de fonctionnement en figure 5.

Le couple pression/température doit être maintenu de telle manière que le milieu ne soit pas en phase gaz.

Le temps de séjour dans ces conditions peut se situer dans une gamme allant de 1 à 60 minutes.

Par la suite, il est considéré un essai à 290°C, et 10 MPa, avec un temps de séjour de 5 minutes. Il va de soi que cela ne constitue qu'un exemple parmi les multitudes de conditions opératoires possibles.

Pour traiter un kilogramme de solution algale, de densité égale à 1 en première approximation, il faut environ 1000 kJ pour chauffer l'eau présente dans la solution algale de 20 à 290°C, et environ 125 kJ pour chauffer la matière organique et inorganique.

Ceci constitue une première approximation de l'énergie à fournir idéalement pour chauffer le milieu réactionnel. A cette valeur s'ajoute l'enthalpie de réaction de liquéfaction. En suivant les estimations de la publication [4], celle-ci peut être estimée à 27 kJ dans ces conditions.

Les conditions opératoires peuvent influer de manière importante sur les résultats.

Ainsi la température, la pression, le temps de séjour, le type et la souche d'algues, leurs concentrations, et leurs procédés de culture, font varier le taux de bio-crude obtenue et sa qualité, ainsi que la nature de l'effluent aqueux et sa concentration en composés carbonés.

### iii. Produits en sortie

Dans les conditions citées précédemment, il est attendu approximativement 7%massique de bio-crude (avec un pouvoir calorifique supérieur avoisinant 32 à 38 MJ/kg), 1%_{massique} de CO₂, supercritique dans ces conditions de température et pression, et 92%massique d'effluent aqueux.

Ce dernier contient 6%massique de carbone organique, soit 54,8 g de carbone par litre de solution algale traitée.

### Etape d'oxydation en voie humide

Lorsqu'un réacteur 7 à double enveloppe est utilisé, cette réaction d'oxydation en voie humide intervient dans l'espace entre les enveloppes internes et externes (ou inversement au sein de la zone interne).

### iv. Produits en entrée

En entrée de réacteur 5, sont introduits les effluents aqueux, issus de la réaction de liquéfaction, ainsi qu'un oxydant de type air ou oxygène.

La phase aqueuse, contenant des composés carbonés et du CO₂, subit ainsi une oxydation en voie humide dans ces conditions de température et pression, grâce à l'ajout d'un oxydant.

En première approximation, les 54,8 g de carbone présents dans les effluents aqueux, peuvent libérer 1800 kJ lors de l'oxydation (suivant la composition élémentaire des composés carbonés la valeur se situera plutôt en réalité entre 1000 et 1800 kJ).

En première approximation cette valeur suffit donc à apporter l'énergie nécessaire, en tant que chaleur à la réaction de liquéfaction hydrothermale et au réchauffement de l'oxydant.

### v. Conditions opératoires

La réaction d'oxydation en voie humide se déroule dans les mêmes conditions que la liquéfaction hydrothermale, *i*.*e*. dans une gamme de températures de 200 à 350°C, de pression comprise entre 5 et 25 MPa. L'ajout d'oxydant doit se faire en quantité sur-stœchiométrique (1,5 par exemple).

Le temps de séjour nécessaire est également de l'ordre de 1 à 60 minutes.

Pour 54,8 g de carbone il faut un minimum théorique de 146,1 g d'oxygène pour assurer une oxydation théorique totale.

En réalité une sur-stœchiométrie est favorable. Par exemple, pour obtenir une sur-stœchiométrie de 1,5, il faut 219,2 g d'oxygène.

Comme l'oxygène arrive à température ambiante, il nécessite 54,5 kJ pour le chauffer à la température de réaction. Si l'oxydant est de l'air, il s'ajouterait 767,6 g d'azote nécessitant 213,3 kJ pour être portés de 20 à 290°C.

### vi. Produits en sortie

En sortie du réacteur 5 d'oxydation, le flux est composé d'un milieu propice à la culture des algues, contenant de l'eau, du CO₂ et les nutriments initialement présents dans la solution algale.

Ces éléments peuvent être injectés directement dans la zone de culture.

D'autres variantes et améliorations peuvent être prévues sans pour autant sortir du cadre de l'invention.

L'invention n'est pas limitée aux exemples qui viennent d'être décrits; il est notamment possible de combiner entre elles des caractéristiques des exemples illustrés au sein de variantes non illustrées.

L'expression « comportant un » doit être comprise comme étant synonyme de « comportant au moins un », sauf si le contraire est spécifié.

### Références citées

[1]: J. Pruvost et Al. « Production industrielle de microalgues et cyanobactéries », Techniques de l'ingénieur, IN 200, 11/2011 ;
[2]: Julia L. Faeth, et Al. : « Fast Hydrothermal Liquefaction of Nannochloropsis sp. To Produce Biocrude », Energy Fuels, 2013, 27 (3), pp 1391-1398;
[3]: Yan Zhou, et Al.: « A synergistic combination of algal wastewater treatment and hydrothermal biofuel production maximized by nutrient and carbon recycling», Energy & Environmental Sciences, 2013, 6, 3765-3779;
[4]: Mariane Audo : « Evaluation du potentiel rhéologique d'huiles issues de microalgues pour des applications en tant que matériaux de substitutions aux bitumes » Thèse de doctorat, 2013, Université de Nantes, école doctorale 3MPL.

## Revendications

1. Procédé de conversion de la biomasse algale en un gaz ou en un bio-crude en vue de produire un combustible ou un carburant, notamment un carburant liquide, ou un autre produit de synthèse, comprenant les étapes suivantes :
a/ gazéification ou liquéfaction hydrothermale d'une biomasse algale dans au moins un premier réacteur (1),
b/ séparation entre respectivement le gaz ou le bio-crude produit, et les effluents aqueux et le CO₂ produit, en sortie du premier réacteur,
c/ récupération des effluents aqueux,
d/ oxydation des effluents aqueux dans au moins un deuxième réacteur,
l'étape d/ étant une oxydation par voie humide ou une oxydation hydrothermale (OHT) en conditions supercritiques,
la chaleur produite lors de l'étape d/ d'oxydation dans le deuxième réacteur étant apportée dans le premier réacteur pour la mise en œuvre de l'étape a/ de liquéfaction ou gazéification hydrothermale.

2. Procédé de conversion selon la revendication 1, l'étape a/ étant une liquéfaction, réalisée dans une gamme de températures comprises entre 150 et 374°C, de préférence réalisée dans une gamme de pressions comprises entre 0,5 et 35 MPa.

3. Procédé de conversion selon la revendication 1, l'étape a/ étant une gazéification, réalisée dans une gamme de températures comprises entre 374 et 800°C, de préférence réalisée dans une gamme de pressions comprises entre 22,1 et 35 MPa.

4. Procédé de conversion selon l'une des revendications précédentes, l'étape d/ étant une oxydation par voie humide réalisée dans une gamme de températures comprises entre 150 et 374°C, de préférence réalisée dans une gamme de pressions comprises entre 0,5 et 35 MPa.

5. Procédé de conversion selon l'une des revendications 1 à 3, l'étape d/ étant une oxydation hydrothermale réalisée dans une gamme de températures comprises entre 374 et 800°C, de préférence réalisée dans une gamme de pressions comprises entre 22,1 et 35 MPa.

6. Procédé de conversion selon l'une des revendications précédentes, l'étape d/ étant une oxydation par voie humide réalisée avec une partie du bio-crude produit injecté dans le deuxième réacteur.

7. Procédé de conversion selon l'une des revendications précédentes, le CO₂ produit et séparé étant injecté, en tant que solvant, dans le bio-crude produit.

8. Procédé de conversion selon l'une des revendications précédentes, la chaleur produite lors de l'étape d/ d'oxydation dans le deuxième réacteur étant apportée pour préchauffer le gaz oxydant.

9. Procédé de conversion selon l'une des revendications précédentes, l'étape a/ de liquéfaction hydrothermale et l'étape d/ d'oxydation étant réalisées au sein d'un même réacteur, dit à double enveloppe, comprenant une enveloppe interne délimitant intérieurement la chambre du premier réacteur, et une enveloppe externe entourant l'enveloppe interne, l'espace entre l'enveloppe interne et l'enveloppe externe définissant la chambre du deuxième réacteur.

10. Procédé de conversion selon l'une des revendications précédentes, l'étape a/ de liquéfaction ou gazéification hydrothermale étant réalisée dans plusieurs premiers réacteurs en parallèle fluidique.

11. Procédé de conversion selon l'une des revendications précédentes, l'étape d/ d'oxydation étant réalisée dans plusieurs deuxièmes réacteurs en parallèle fluidique.

12. Procédé en continu de culture de biomasse algale et de conversion de la biomasse algale cultivée en un gaz ou en un bio-crude, comprenant les étapes suivantes :
- culture de la biomasse algale dans une zone contenant un milieu de culture
- récolte de la biomasse algale cultivée,
- étapes a/ à d/ du procédé de conversion selon l'une des revendications précédentes,
- injection de l'eau et des nutriments, et le cas échéant du CO₂, obtenus en sortie du deuxième réacteur, dans la zone de culture.

13. Procédé en continu selon la revendication 12, comprenant en outre l'étape de récupération de l'oxygène (O₂) produit par la biomasse algale pour l'injecter, en tant qu'oxydant, en amont du deuxième réacteur avant l'étape d/ d'oxydation.

## Patentansprüche

1. Verfahren zur Umwandlung von Algenbiomasse zu einem Gas oder zu einem Bio-Rohöl, um einen Brennstoff oder einen Kraftstoff, insbesondere einen flüssigen Kraftstoff, oder ein anderes Syntheseprodukt herzustellen, umfassend die folgenden Schritte:
a/ hydrothermale Vergasung oder Verflüssigung einer Algenbiomasse in mindestens einem ersten Reaktor (1),
b/ Trennung zwischen dem erzeugten Gas bzw. Bio-Rohöl und dem Abwasser und dem erzeugten CO₂ im Auslass des ersten Reaktors,
c/ Rückführen des Abwassers,
d/ Oxidation des Abwassers in mindestens einem zweiten Reaktor,
wobei Schritt d/ eine Nassoxidation oder eine hydrothermale Oxidation (OHT) unter superkritischen Bedingungen ist,
wobei die beim Oxidationsschritt d/ im zweiten Reaktor erzeugte Wärme zur Durchführung des Schritts a/ der hydrothermalen Verflüssigung oder Vergasung in den ersten Reaktor geführt wird.

2. Umwandlungsverfahren nach Anspruch 1, wobei Schritt a/ eine Verflüssigung ist, die in einem Temperaturbereich zwischen 150 und 374 °C ausgeführt wird, bevorzugt in einem Druckbereich zwischen 0,5 und 35 MPa ausgeführt wird.

3. Umwandlungsverfahren nach Anspruch 1, wobei Schritt a/ eine Vergasung ist, die in einem Temperaturbereich zwischen 374 und 800 °C ausgeführt wird, bevorzugt in einem Druckbereich zwischen 22,1 und 35 MPa ausgeführt wird.

4. Umwandlungsverfahren nach einem der vorhergehenden Ansprüche, wobei Schritt d/ eine Nassoxidation ist, die in einem Temperaturbereich zwischen 150 und 374 °C ausgeführt wird, bevorzugt in einem Druckbereich zwischen 0,5 und 35 MPa ausgeführt wird.

5. Umwandlungsverfahren nach einem der Ansprüche 1 bis 3, wobei Schritt d/ eine hydrothermale Oxidation ist, die in einem Temperaturbereich zwischen 374 und 800 °C ausgeführt wird, bevorzugt in einem Druckbereich zwischen 22,1 und 35 MPa ausgeführt wird.

6. Umwandlungsverfahren nach einem der vorhergehenden Ansprüche, wobei Schritt d/ eine Nassoxidation ist, die mit einem Teil des erzeugten Bio-Rohöls ausgeführt wird, das in den zweiten Reaktor eingespritzt wird.

7. Umwandlungsverfahren nach einem der vorhergehenden Ansprüche, wobei das erzeugte und getrennte CO₂ als Lösungsmittel in das erzeugte Bio-Rohöl eingespritzt wird.

8. Umwandlungsverfahren nach einem der vorhergehenden Ansprüche, wobei die beim Oxidationsschritt d/ im zweiten Reaktor erzeugte Wärme zugeführt wird, um das oxidierende Gas vorzuwärmen.

9. Umwandlungsverfahren nach einem der vorhergehenden Ansprüche, wobei Schritt a/ der hydrothermalen Verflüssigung und der Oxidationsschritt d/ in demselben Reaktor, Doppelmantelreaktor genannt, ausgeführt werden, der einen Innenmantel umfasst, der innen die Kammer des ersten Reaktors begrenzt, und einen Außenmantel, der den Innenmantel umgibt, wobei der Raum zwischen dem Innenmantel und dem Außenmantel die Kammer des zweiten Reaktors definiert.

10. Umwandlungsverfahren nach einem der vorhergehenden Ansprüche, wobei Schritt a/ der hydrothermalen Verflüssigung oder Vergasung in mehreren ersten Reaktoren fluidisch parallel ausgeführt wird.

11. Umwandlungsverfahren nach einem der vorhergehenden Ansprüche, wobei der Oxidationsschritt d/ in mehreren zweiten Reaktoren fluidisch parallel ausgeführt wird.

12. Kontinuierliches Verfahren zur Kultivierung von Algenbiomasse und zur Umwandlung der kultivierten Algenbiomasse zu einem Gas oder zu einem Bio-Rohöl, umfassend die folgenden Schritte:
- Kultivierung der Algenbiomasse in einem Bereich, der ein Kulturmedium enthält,
- Ernten der kultivierten Algenbiomasse,
- die Schritte a/ bis d/ des Umwandlungsverfahrens nach einem der vorhergehenden Ansprüche,
- Einspritzen des Wassers und der Nährstoffe und gegebenenfalls des CO₂, die im Auslass des zweiten Reaktors erhalten werden, in den Kultivierungsbereich.

13. Kontinuierliches Verfahren nach Anspruch 12, umfassend ferner den Schritt der Rückführung des von der Algenbiomasse erzeugten Sauerstoffs (O₂), um ihn als Oxidationsmittel vor dem Oxidationsschritt d/ stromauf des zweiten Reaktors einzuspritzen.

## Claims

1. A method for converting algal biomass into a gas or into a biocrude with a view to producing a fuel or a motor fuel, notably a liquid fuel, or some other synthetic product, comprising the following steps:
a/ gasification or hydrothermal of an algal biomass in at least one first reactor (1),
b/ separation between respectively the gas or biocrude produced, and the aqueous effluents and the CO₂ produced, at the outlet of the first reactor,
c/ recovery of the aqueous effluents,
d/ oxidation of the aqueous effluents in at least one second reactor.
step d/ being an oxidation by a wet process or a hydrothermal oxidation (HTO) in supercritical conditions,
the heat produced during step d/ of oxidation in the second reactor being supplied to the first reactor for carrying out step a/ of hydrothermal liquefaction or gasification.

2. The method of conversion as claimed in claim 1, step a/ being a liquefaction, carried out in a temperature range between 150 and 374°C, preferably being a liquefaction, carried out in a range of pressures between 0.5 and 35 MPa.

3. The method of conversion as claimed in claim 1, step a/ being a gasification, carried out in a temperature range between 374 and 800°C, preferably being a gasification, carried out in a range of pressures between 22.1 and 35 MPa.

4. The method of conversion as claimed in one of the preceding claims, step d/ being a wet-process oxidation carried out in a temperature range between 150 and 374°C, preferably being a wet-process oxidation carried out in a range of pressures between 0.5 and 35 MPa.

5. The method of conversion as claimed in claims 1 to 3, step d/ being a hydrothermal oxidation carried out in a temperature range between 374 and 800°C, preferably being a hydrothermal oxidation carried out in a range of pressures between 22.1 and 35 MPa.

6. The method of conversion as claimed in one of the preceding claims, step d/ being a wet-process oxidation carried out with part of the biocrude produced injected into the second reactor.

7. The method of conversion as claimed in one of the preceding claims, the CO₂ produced and separated being injected, as solvent, in the biocrude product.

8. The method of conversion as claimed in one of the preceding claims, the heat produced during step d/ of oxidation in the second reactor being supplied for preheating the oxidizing gas.

9. The method of conversion as claimed in one of the preceding claims, step a/ of hydrothermal liquefaction and step d/ of oxidation being carried out in one and the same reactor, called a double-jacketed reactor, comprising an inner jacket delimiting the chamber of the first reactor internally, and an outer jacket surrounding the inner jacket, with the space between the inner jacket and the outer jacket defining the chamber of the second reactor.

10. The method of conversion as claimed in one of the preceding claims step a/ of hydrothermal liquefaction or gasification being carried out in several first reactors fluidically in parallel.

11. The method of conversion as claimed in one of the preceding claims, step d/ of oxidation being carried out in several second reactors fluidically in parallel.

12. A continuous process for culture of algal biomass and conversion of the algal biomass cultivated into a gas or into a biocrude, comprising the following steps:
- culture of the algal biomass in a zone containing a culture medium
- harvesting the algal biomass cultivated,
- steps a/ to d/ of the method of conversion as claimed in one of the preceding claims,
- injecting the water and the nutrients, and if applicable the CO₂, obtained at the outlet of the second reactor, into the culture zone.

13. The continuous process as claimed in claim 12, further comprising the step of recovery of the oxygen (O₂) produced by the algal biomass, to inject it, as oxidant, upstream of the second reactor before the oxidation step d/.
